# EUROPÄISCHE PATENTSCHRIFT

(11) **EP 3 493 557 B1**
(45) Veröffentlichungstag und Bekanntmachung des Hinweises auf die Patenterteilung: **12.05.2021**
(21) Anmeldenummer: 18204856.1
(22) Anmeldetag: 07.11.2018
(51) Int. Cl.: H04R 25/00, A61M 21/00, G10K 11/175

(54) **VERFAHREN ZUM BETREIBEN EINER VORRICHTUNG ZUR TINNITUS-CHARAKTERISIERUNG SOWIE ENTSPRECHENDE VORRICHTUNG**
METHOD FOR OPERATING A DEVICE FOR TINNITUS CHARACTERISATION AND CORRESPONDING DEVICE
PROCÉDÉ DE FONCTIONNEMENT D'UN DISPOSITIF DE CARACTÉRISATION DE TINNITUS AINSI QUE DISPOSITIF CORRESPONDANT

(30) Priorität: 30.11.2017 DE 102017221611
(43) Veröffentlichungstag der Anmeldung: 05.06.2019
(73) Patentinhaber: Sivantos Pte. Ltd., Singapore 539775 (SG)
(72) Erfinder: MÜLLER-WEHLAU, Matthias, 90411 Nürnberg (DE)
(74) Vertreter: FDST Patentanwälte

(56) Entgegenhaltungen:
- DE-A1- 10 128 642
- DE-A1-102007 046 020
- DE-A1-102012 220 620

## Beschreibung

Die Erfindung betrifft ein Verfahren zum Betreiben einer Vorrichtung zur Tinnitus-Charakterisierung und eine entsprechende Vorrichtung.

Ein schwerer Hörverlust als Hörschädigung löst häufig eine neuroplastische Reorganisation des zentralen auditorischen Systems im Gehirn des Betroffenen aus, und ist daher häufig ein Auslöser und eine Ursache eines auftretenden (chronischen) Tinnitus.

Unter einem Tinnitus oder Ohrensausen versteht man allgemein alle Arten von Kopf- oder Ohrgeräuschen, welche nicht durch ins Ohr geführte akustische Signale der Umgebung bewirkt werden. Ein Tinnitus ist zwar ungefährlich, belastet jedoch viele Betroffene stark. Beispielsweise werden die Konzentration gestört und Einschlafprobleme verursacht. So führt insbesondere chronischer Tinnitus oftmals zu schweren psychologischen Problemen und wirkt sich somit mitunter negativ auf das berufliche und soziale Leben der betroffenen Person aus.

Im Zuge einer Tinnitus-Therapie werden häufig Rauschgeräte (Tinnitus-Noiser, Audiostimulator, Tinnitus Control Instrument, Tinnitus-Masker) verwendet. Hierzu wird dem Betroffenen mit Hilfe einer Hörhilfevorrichtung ähnlichen Tinnitus-Therapie-Vorrichtung, einem sogenannten Noiser oder Masker, ein leises, wenig störendes Geräusch als akustisches Signal angeboten, welches den Tinnitus überdecken ("maskieren") oder als Ablenkung dienen soll.

Beispielsweise sind auch sogenannte Notch-Therapien möglich, bei welchen ein akustisches Signal der Umgebung für den Betroffenen erzeugt wird, in dem eine oder mehrere der Tinnitusfrequenzen des wahrgenommenen Tinnitusgeräuschs unterdrückt sind. Dadurch wird eine neuroplastische Reorganisation des zentralen auditorischen Systems des Betroffenen ermöglicht, welche die tinnitusverursachende maladaptive neuroplastische Reorganisation des zentralen auditorischen Systems des Betroffenen wieder rückgängig macht.

Für eine effektive Therapie oder Behandlung ist es jedoch stets notwendig, dass das vom Betroffenen wahrgenommene Tinnitusgeräusch, also die oder jede Tinnitusfrequenz sowie deren jeweilige Amplitude oder Intensität, möglichst exakt identifiziert und charakterisiert wird.

Zur Charakterisierung des Tinnitusgeräuschs werden in der Regel Tinnitus-Analysen (Tinnitus-Matching) durchgeführt. Hierzu sind beispielsweise Vorrichtungen zur Tinnitus-Charakterisierung denkbar, die einen Signalgenerator zur Erzeugung von Audiosignalen beziehungsweise akustischen Testtönen aufweist, welche beispielsweise mittels eines Kopfhörers dem Betroffenen eingespielt werden.

Bei einer Tinnitus-Analyse beziehungsweise bei einem Tinnitus-Matching werden dem Betroffenen in der Regel zwei (Test-)Töne dargeboten, deren jeweilige Ton- oder Signalfrequenzen einen vorgebenden Frequenzabstand zueinander aufweisen. Der Betroffene vergleicht die (externen) Töne mit seinem wahrgenommenen (internen) Tinnitusgeräusch, wobei in Abhängigkeit des Vergleichs zwei neue Töne mit zwei neuen Tonfrequenzen erzeugt werden. Die Frequenzschrittweite der neuen Töne wird hierbei mit jedem Vergleich sukzessive reduziert, sodass sich die Töne mit jedem Vergleich weiter an das wahrgenommene Tinnitusgeräusch beziehungsweise an die oder jede Tinnitusfrequenz sukzessive annähern.

Ein derartiges Tinnitus-Matching mittels Vergleichsmessungen weist jedoch einige Nachteile auf. So ist es insbesondere für ungeübte Zuhörer häufig schwierig, die dargebotenen Testtöne zuverlässig mit dem wahrgenommenen Tinnitusgeräusch zu vergleichen. Dadurch werden die Reproduzierbarkeit sowie die Genauigkeit verschlechtert, sodass eine vergleichsweise hohe Variabilität der Matching-Ergebnisse auftritt. Insbesondere tritt hierbei häufig das Problem einer sogenannten Oktavenkonfusion auf. Dies bedeutet, dass die vom Betroffenen bestimmte Tinnitusfrequenz von der richtigen Tinnitusfrequenz um eine Oktave zu höheren oder niedrigeren Tönen abweicht. Dies tritt insbesondere bei Tinnitusgeräuschen auf, welche nicht lediglich aus einer einzelnen (reinen) Ton- beziehungsweise Tinnitusfrequenz bestehen, sondern eine Anzahl von unterschiedlichen Tinnitusfrequenzen aufweisen.

In Abhängigkeit von der Anzahl an Vergleichen der erzeugten Testtöne mit dem Tinnitusgeräusch ist die Frequenzschrittweite, und somit die Frequenzauflösung, dadurch beschränkt, dass der Betroffene sein Tinnitusgeräusch zuverlässig identifiziert und den jeweiligen Tönen korrekt zuordnet. Beispielsweise benötigen Notch-Therapien eine vergleichsweise hohe Frequenzauflösung der oder jeder Tinnitusfrequenz, sodass ein solches Tinnitus-Matching besonders zeitaufwändig wird.

Zusätzlich zu der Bestimmung der oder jeder Tinnitusfrequenz ist es typischerweise notwendig, dass die Amplitude der oder jeder Tinnitusfrequenz im wahrgenommenen Tinnitusgeräusch bestimmt wird. Häufig wird hierzu am Anfang des Tinnitus-Matchings die Lautstärke der erzeugten (Test-)Töne angepasst, bis sie in etwa der Lautstärke des wahrgenommenen Tinnitusgeräuschs entspricht. Da diese Lautstärkeanpassung jedoch in der Regel bei einer von der oder jeder Tinnitusfrequenz abweichenden (Ton-)Frequenz erfolgt, ist es nicht gewährleistet, dass die bestimmte Amplitude der tatsächlichen Amplitude an der oder jeder Tinnitusfrequenz entspricht. Insbesondere bei Betroffenen mit einem zusätzlichen Hörverlust treten hierbei häufig wesentliche Abweichungen zwischen den bestimmten Amplituden und den tatsächlichen Amplituden des Tinnitusgeräuschs auf.

Die DE 10 2007 046 020 A1 offenbart eine Vorrichtung zur Analyse und Synthese von Audiosignalen. Die Vorrichtung weist einen Speicherabschnitt, in dem eine Mehrzahl von Basissignalen speicherbar und/oder gespeichert ist, und einen Ausgabeabschnitt, mit dem Audiosignale akustisch reproduzierbar sind, sowie einen Auswahlabschnitt, mit dem aus von mit dem Ausgabeabschnitt ausgegebenen Audiosignalen eines der ausgegebenen Audiosignale als ein Auswahlsignal auswählbar ist, und eine Analyse- und Syntheseeinheit, mit der basierend auf einem mit dem Auswahlabschnitt ausgewählten Auswahlsignal jeweils durch Zusammensetzung mehrerer Basissignale Kombinationssignale generierbar und zu ihrer akustischen Reproduktion an den Ausgabeabschnitt übermittelbar sind, auf. Mit der Analyse- und Syntheseeinheit ist mindestens ein erstes Kombinationssignal generierbar und mittels des Auswahlabschnittes als Analysesignal auswählbar, wobei basierend auf dem Analysesignal oder einem davon abgeleiteten Signal mit der Analyse- und Syntheseeinheit mindestens ein zweites Kombinationssignal generierbar und mittels des Auswahlabschnittes als Synthesesignal auswählbar ist.

In der DE 101 28 642 A1 sind ein medizintechnisches Verfahren und eine Anordnung zur Unterdrückung von Ohrgeräuschen (Tinnitus) durch den Einsatz von Kompensationsschall beschrieben. Die Anordnung besteht aus einer Anzahl von NF-Generatoren, einer Mischeinrichtung, einem Verstärker und einem Schallwandler, welche sowohl elektrisch als auch über eine spezielle Software mit einer Steuer- und Auswerteeinheit verbunden sind.

Der Erfindung liegt die Aufgabe zugrunde, ein besonders geeignetes Verfahren zum Betreiben einer Vorrichtung zur Tinnitus-Charakterisierung anzugeben. Insbesondere soll es einem Betroffenen erleichtert werden, zuverlässig das Tinnitusgeräusch zu bestimmen. Der Erfindung liegt weiterhin die Aufgabe zugrunde, eine für ein derartiges Verfahren geeignete Vorrichtung anzugeben.

Hinsichtlich des Verfahrens wird die Aufgabe mit den Merkmalen des Anspruchs 1 und hinsichtlich der Vorrichtung mit den Merkmalen des Anspruchs 6 erfindungsgemäß gelöst. Vorteilhafte Ausgestaltungen und Weiterbildungen sind Gegenstand der jeweiligen Unteransprüche.

Das erfindungsgemäße Verfahren ist zum Betreiben einer Vorrichtung zur Tinnitus-Charakterisierung geeignet und ausgestaltet.

Verfahrensgemäß wird in einem ersten Verfahrensschritt ein breitbandiges Testsignal mit einer Anzahl von Signalfrequenzen als akustisches Signal beziehungsweise Audiosignal erzeugt und mit einem Tinnitusgeräusch verglichen. Für jedes dargebotene Testsignal wird ein Vergleich mit dem Tinnitusgeräusch durchgeführt, und beurteilt, ob das Testsignal den wahrgenommenen Tinnitus maskiert oder nicht. Das jeweilige Testsignal wird zusammen mit dem zugeordneten Vergleichsergebnis hinterlegt. Hierbei werden insbesondere sowohl positive Vergleichsergebnisse, bei welchen das Tinnitusgeräusch von dem Testsignal maskiert wird, als auch negative Vergleichsergebnisse, bei welchen das Testsignal den Tinnitus nicht oder lediglich teilweise maskiert, ausgewertet.

Aus den hinterlegten Vergleichsergebnissen und Testsignalen wird eine Wahrscheinlichkeits-Korrelation (likelihood correlation) zur Ermittlung des Tinnitusgeräuschs bestimmt. Dies bedeutet, dass aus den Testsignalen in Abhängigkeit der Maskierungswirkung die Wahrscheinlichkeits-Korrelation bestimmt wird. Mit anderen Worten wird im ersten Verfahrensschritt eine Verdeckungsmessung des Tinnitusgeräuschs durchgeführt, wobei aus den Verdeckungs- oder Maskierungseigenschaften der Testsignale die Wahrscheinlichkeits-Korrelation abgeleitet wird.

In einem zweiten Verfahrensschritt werden die Amplituden der einzelnen Signalfrequenzen des Testsignals variiert. Der erste und zweite Verfahrensschritt werden wiederholt durchgeführt, bis die Wahrscheinlichkeits-Korrelation einen ersten Schwellwert erreicht oder überschreitet. Mit anderen Worten wird das Testsignal verändert und anschließend erneut mit dem Tinnitusgeräusch verglichen. Dies wird sukzessive wiederholt, und somit weitere Vergleichsergebnisse erzeugt, bis die Wahrscheinlichkeits-Korrelation den ersten Schwellwert erreicht oder überschreitet. Der erste Schwellwert entspricht hierbei im Wesentlichen einer Mindestanzahl von insbesondere positiven Vergleichsergebnissen beziehungsweise hinterlegten Testsignalen, welche zur Bestimmung einer geeigneten Wahrscheinlichkeits-Korrelation benötigt werden.

Ist der erste Schwellwert erreicht oder überschritten werden in einem dritten Verfahrensschritt die Amplituden der einzelnen Signalfrequenzen des Testsignals anhand der Wahrscheinlichkeits-Korrelation variiert. Mit anderen Worten wird anhand der Vergleichsergebnisse oder Maskierungswirkungen ein Testsignal geschätzt, welches das Tinnitusgeräusch plausibel reproduziert, wobei mit zunehmender Anzahl an Vergleichsergebnissen die Plausibilität zunehmend verbessert wird. Die Bestimmung einer möglichst plausiblen Wahrscheinlichkeits-Korrelation entspricht somit im Wesentlichen der Charakterisierung des Tinnitusgeräuschs. Dadurch ist ein besonders geeignetes Verfahren zum Betreiben einer Vorrichtung zur Tinnitus-Charakterisierung realisiert.

Somit wird die vergleichsweise schwierige Aufgabe der Identifizierung einzelner Tinnitusfrequenzen dahingehend vereinfacht, dass ein breitbandiges Testsignal erzeugt wird, und der Betroffene dieses Testsignal hinsichtlich einer Maskierung oder Überdeckung beziehungsweise Verdeckung seines wahrgenommenen Tinnitusgeräuschs vergleicht. Mit anderen Worten bewertet der Betroffene, ob er während der Einspielung beziehungsweise Darbietung des Testsignals sein Tinnitusgeräusch wahrnimmt.

Im Gegensatz zum Stand der Technik vergleicht der Betroffene somit nicht einzelne (Ton-)Frequenzen miteinander, sondern bewertet lediglich ob eine Maskierung durch das Testsignal bewirkt wird oder nicht. Dies bedeutet, dass anstelle einer Vergleichsmessung eine Verdeckungsmessung durchgeführt wird, wodurch insbesondere die Gefahr einer Oktavenkonfusion im Wesentlichen vollständig vermieden wird. Dadurch wird eine besonders geeignete Tinnitus-Charakterisierung auch für ungeübte Zuhörer realisiert.

Durch die sukzessive Erzeugung neuer (veränderter) Testsignale ist es mittels der daraus bestimmbaren Wahrscheinlichkeits-Korrelation möglich, das Tinnitusgeräusch, also die oder jede Tinnitusfrequenz, sowie deren jeweilige Amplitude, zuverlässig zu bestimmen.

Die breitbandigen Testsignale umfassen hierbei jeweils eine Anzahl von Signalfrequenzen. Dies bedeutet, dass die Testsignale aus einer Anzahl von (spektral) aneinandergereihten, schmalbandigen oder tonalen Geräuschkomponenten zusammengesetzt sind.

Das Testsignal wird dem Betroffenen geeigneterweise ausreichend lang eingespielt beziehungsweise dargeboten, sodass dieser das Testsignal mit dem Tinnitusgeräusch zuverlässig vergleichen kann. Mit anderen Worten überprüft der Betroffene, ob er sein Tinnitusgeräusch in der Gegenwart des Testsignals wahrnehmen kann. Nach der Antwort des Betroffenen werden die Amplituden der einzelnen Signalfrequenzen beziehungsweise Schmalbandgeräusche des Testsignals variiert. Aus den Vergleichsergebnissen wird die Wahrscheinlichkeits-Korrelation bestimmt, wobei diese im dritten Verfahrensschritt zur gezielten Einstellung oder Variierung der Schmalbandgeräusche verwendet wird. Dadurch wird eine schnelle und zuverlässige Bestimmung des Tinnitusgeräuschs gewährleistet.

In einer vorteilhaften Ausführung werden der erste und dritte Verfahrensschritt mehrmals wiederholt, bis die Wahrscheinlichkeits-Korrelation einen zweiten Schwellwert erreicht oder überschreitet. Der zweite Schwellwert ist hierbei geeigneterweise eine Mindestwahrscheinlichkeit beziehungsweise ein Wahrscheinlichkeitsmaß, dass das aus der Wahrscheinlichkeits-Korrelation abgeleitete Testsignal dem tatsächlichen Tinnitusgeräusch entspricht. Erreicht oder Überschreitet die Wahrscheinlichkeits-Korrelation den zweiten Schwellwert, wird das Verfahren beendet und das aus der Wahrscheinlichkeits-Korrelation bestimmbare Testsignal als ein dem Tinnitusgeräusch entsprechendes akustisches Signal ausgegeben und/oder in einem Speicher hinterlegt. Dadurch ist eine besonders effektive Tinnitus-Charakterisierung realisiert.

In einer geeigneten Weiterbildung werden die Amplituden der einzelnen Signalfrequenzen beziehungsweise Schmalbandgeräusche des Testsignals im zweiten Verfahrensschritt zufällig eingestellt. Geeigneterweise wird zu Beginn des Verfahrens, das bedeutet bei dem ersten Vergleich, ein Testsignal mit zufälligen Amplitudenwerten der einzelnen Signalfrequenzen verwendet. Dadurch ist dem Umstand Rechnung getragen, dass das Tinnitusgeräusch am Anfang des Verfahrens im Wesentlichen unbekannt ist. Durch die zufällige Amplitudenverteilung der Signalfrequenzen ist eine besonders effektive Bestimmung der Wahrscheinlichkeits-Korrelation und somit des Tinnitusgeräuschs ermöglicht.

In einer zweckmäßigen Ausgestaltung des Verfahrens wird zur Bestimmung der Wahrscheinlichkeits-Korrelation ein physiologisches Modell eines Ohrs beziehungsweise eines Abschnittes oder Teils des Ohrs verwendet. Unter einem physiologischen Modell ist hierbei insbesondere ein mathematisches und/oder computer- beziehungsweise programmtechnisches Modell zur Beschreibung oder Simulation einer physiologischen Komponente des Ohrs beziehungsweise einer physiologischen Funktion einer derartigen Komponente zu verstehen. Mit anderen Worten ermöglicht das physiologische Modell physiologische Eigenschaften oder Reaktionen des Ohrs zu bestimmen. Dadurch ist ein besonders geeignetes Verfahren realisiert. Als Eingangsgrößen werden hierbei insbesondere die Signalfrequenzen und deren Amplituden derjenigen Testsignale verwendet, die ein positives Vergleichsergebnis aufweisen.

In einer bevorzugten Ausbildung wird als physiologisches Modell ein Modell einer Basilarmembran (Lamina basilaris oder Membrana basilaris) verwendet, wobei die Wahrscheinlichkeits-Korrelation anhand eines Anregungsmusters der modellierten Basilarmembran bestimmt wird.

Die Tonhöhe, die bei einer Beschallung mit einem akustischen Signal einer gewissen Frequenz von einem Betroffenen wahrgenommen wird, hängt eng mit dem Ort auf der Basilarmembran zusammen, an dem bei dieser Frequenz ein Erregungsmaximum besteht. Das durch ein akustisches Signal einer bestimmten ersten Frequenz bewirkte Erregungsmuster ist dabei nicht scharf in einem Bereich der Basilarmembran lokalisiert, sondern führt auch in hierzu räumlich benachbarten Bereichen der Basilarmembran zu einer Anregung oder Erregung. Diese räumlich ausgedehnte Anregung führt zu dem Effekt einer auditorischen Maskierung oder spektralen Verdeckung, bei welcher ein Ton einer zweiten Frequenz nicht wahrgenommen werden kann, wenn die durch diesen Ton hervorgerufene Anregung auf der Basilarmembran geringer ist als die eines benachbarten Tons höherer Intensität.

Durch die Bestimmung eines Anregungs- oder Erregungsmusters einer modellierten Basilarmembran ist es somit möglich, eine besonders geeignete und zuverlässige Wahrscheinlichkeits-Korrelation zu bestimmen. Insbesondere ist es somit möglich, physiologische Faktoren, insbesondere auditorische Maskierungen, bei welcher die Anwesenheit eines ersten Tons einen zweiten leiseren Ton nicht mehr wahrnehmbar macht, zur Bestimmung des Tinnitusgeräuschs zu berücksichtigen.

Dies bedeutet, dass für jedes Vergleichsergebnis ein Anregungsmuster berechnet und mit den Anregungsmustern vorangegangener Vergleichsergebnisse korreliert wird. Die dadurch bestimmbaren Gemeinsamkeiten der Anregungsmuster ermöglichen eine besonders zuverlässige Bestimmung der oder jeder Tinnitusfrequenz sowie deren jeweilige (Tinnitus-)Amplitude.

Bei der Modellierung der Basilarmembran und/oder bei der Bestimmung des Anregungs- oder Erregungsmusters ist es möglich, einen gegebenenfalls vorhandenen Hörverlust oder Hörschaden des Betroffenen zu berücksichtigen. Mit anderen Worten ist es möglich, Hörschwellen des Betroffenen bei der Modellierung der Basilarmembran zu verwenden, wodurch die Genauigkeit der Wahrscheinlichkeits-Korrelation, und somit die Bestimmung des Tinnitusgeräuschs, weiter verbessert wird.

In einer bevorzugten Anwendung wird das vorstehend beschriebene Verfahren zum Betrieb einer Vorrichtung zur Tinnitus-Charakterisierung verwendet. Die Vorrichtung umfasst einen Signalgenerator zur Erzeugung eines akustischen Testsignals, sowie einen Hörer, beispielsweise einen Kopfhörer, mittels welchen das Testsignal einem Betroffenen darbietbar beziehungsweise ausgebbar ist. Die Vorrichtung weist hierbei einen Controller (das heißt ein Steuergerät) auf.

Der Controller ist hierbei allgemein - programm- und/oder schaltungstechnisch - zur Durchführung des vorstehend beschriebenen erfindungsgemäßen Verfahrens eingerichtet. Der Controller ist somit insbesondere dazu eingerichtet, anhand der Antworten des Betroffenen, also anhand der Vergleichsergebnisse, das Testsignal zu variieren und eine Wahrscheinlichkeits-Korrelation, insbesondere durch Bestimmung eines Anregungsmuster anhand eines physiologischen Modells einer Basilarmembran, zu bestimmen.

Der Controller ist zumindest im Kern durch jeweils einen Mikrocontroller mit einem Prozessor und einem Datenspeicher gebildet, in dem die Funktionalität zur Durchführung des erfindungsgemäßen Verfahrens in Form einer Betriebssoftware (Firmware) programmtechnisch implementiert ist, so dass das Verfahren - gegebenenfalls in Interaktion mit einem Benutzer - bei Ausführung der Betriebssoftware in dem Mikrocontroller automatisch durchgeführt wird.

Der Controller ist in einer möglichen Ausführungsform im Rahmen der Erfindung alternativ aber auch durch programmierbare elektronische Bauteile, zum Beispiel einen anwendungsspezifischen integrierten Schaltkreis (ASIC) gebildet, in dem die Funktionalität zur Durchführung des erfindungsgemäßen Verfahrens mit schaltungstechnischen Mitteln implementiert ist.

Dadurch ist eine besonders geeignete Vorrichtung realisiert. Durch das erfindungsgemäße Verfahren wird es einem ungeübten Zuhörer beim Benutzen der Vorrichtung erleichtert zuverlässig und mit hoher Genauigkeit das wahrgenommene Tinnitusgeräusch zu ermitteln. Durch die Auswertung der Wahrscheinlichkeit anhand eines physiologischen Anregungsmusters der modellierten Basilarmembran ist es weiterhin möglich, breitbandige Testsignale zu verwenden und hieraus sowohl die einzelnen Tinnitusfrequenzen sowie deren Amplituden des Tinnitusgeräuschs zuverlässig zu bestimmen.

Nachfolgend ist ein Ausführungsbeispiel der Erfindung anhand einer Zeichnung näher erläutert. Darin zeigen:
- Fig. 1: in schematischer und vereinfachter Darstellung eine Vorrichtung zur Tinnitus-Charakterisierung,
- Fig. 2: in einem Flussdiagramm ein Verfahren zum Betreiben der Vorrichtung,
- Fig. 3: in einem Frequenz-Amplituden-Diagramm ein erstes Testsignal und ein Tinnitusgeräusch sowie ein erstes Anregungsmuster, und
- Fig. 4: in einem Frequenz-Amplituden-Diagramm ein zweites Testsignal und das Tinnitusgeräusch sowie ein zweites Anregungsmuster.

Einander entsprechende Teile und Größen sind in allen Figuren stets mit den gleichen Bezugszeichen versehen.

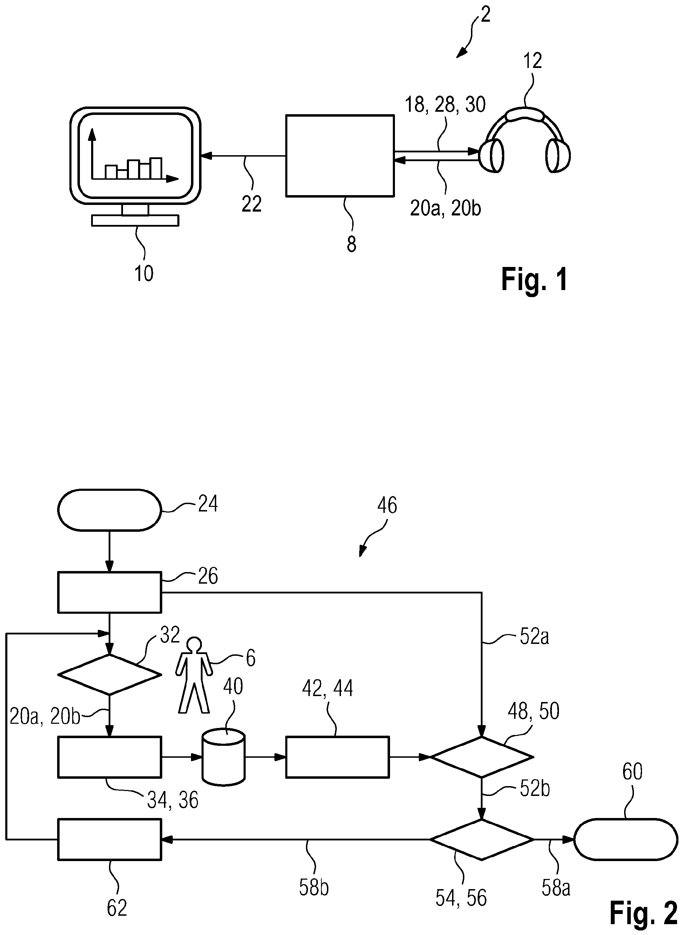

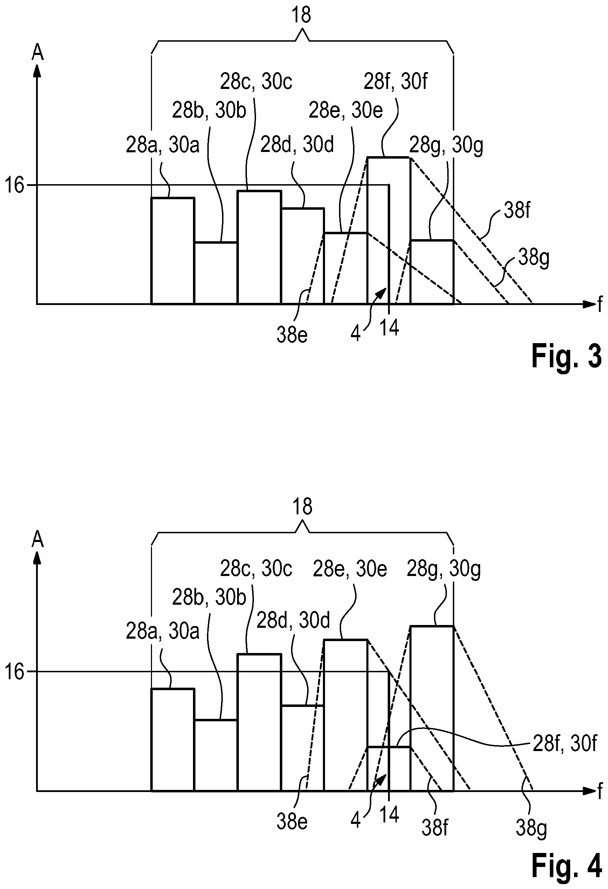

Die Fig. 1 zeigt in vereinfachter und schematisierter Darstellung eine Vorrichtung 2 zur Tinnitus-Charakterisierung, also zur Ermittlung eines Tinnitusgeräuschs 4 (Fig. 3, Fig. 4) eines Betroffenen 6 (Fig. 2). Die Vorrichtung 2 umfasst im Wesentlichen einen Controller 8 als Steuergerät und eine Anzeigeeinheit 10 sowie einen Kopfhörer 12.

Im Zuge der Tinnitus-Charakterisierung wird das Tinnitusgeräusch 4 (Fig. 3, Fig. 4) des Betroffenen 6 möglichst exakt hinsichtlich einer oder jeder wahrgenommenen Tinnitusfrequenz 14 und deren jeweils zugeordneten (Tinnitus-)Amplitude 16 bestimmt. Hierzu setzt der Betroffene 6 den Kopfhörer 12 auf, sodass er während der Charakterisierung möglichst keine akustischen Signale aus der Umgebung wahrnimmt. Mittels eines mit dem Controller 8 gesteuerten Signalgenerators wird ein breitbandiges Testsignal 18 erzeugt und als akustisches Signal beziehungsweise Audiosignal dem Betroffenen 6 mittels des Kopfhörers 12 eingespielt.

Der Betroffene 6 vergleicht das dargebotene Testsignal 18 mit seinem wahrgenommenen Tinnitusgeräusch 4, wobei das Testsignal 18 in Abhängigkeit eines jeweiligen Vergleichsergebnisses 20a, 20b variiert wird. Das Spektrum des Testsignals 18 sowie die Vergleichsergebnisse 20a, 20b werden beispielsweise als Bilddaten 22 an die Anzeigeeinheit 10 übermittelt und einem Benutzer, insbesondere einem HNO-Facharzt oder einem Hörgeräteakustiker, dargestellt. Beispielsweise ist es ebenso denkbar, dass der Betroffene 6 selbst die Vorrichtung 2 bedient und das Verfahren durchführt.

In der Fig. 2 ist anhand eines Flussdiagramms ein Verfahren zum Betreiben der Vorrichtung 2 dargestellt und nachfolgend näher erläutert.

Nach einem Start 24 des Verfahrens wird in einem Verfahrensschritt 26 das breitbandige Testsignal 18 erzeugt. Das Testsignal 18 weist - wie insbesondere in den Figuren 3 und 4 dargestellt - eine Anzahl von hinsichtlich der Frequenz aneinandergereihten Signalfrequenzen oder Schmalbandsignalen 28 auf. In dem Ausführungsbeispiel der Figuren 3 und 4 weist das Testsignal 18 beispielsweise sieben Schmalbandsignale 28 auf. Die Schmalbandsignale 28 beziehungsweise das Testsignal 18 werden hierbei geeigneterweise in einem Frequenzbereich des Tinnitusgeräuschs 4 erzeugt.

Jedes Schmalbandsignal 28 weist hierbei eine jeweilige (Signal-)Amplitude 30 auf, wobei im Verfahrensschritt 26 die Amplituden 30 der Schmalbandsignale 22 zufällig eingestellt werden. Mit anderen Worten weist der Controller 8 beispielsweise einen Zufallszahlengenerator auf, mittels welchen die Amplituden 30 im Verfahrensschritt 26 variiert werden.

Das Testsignal 18 wird in einem anschließenden Vergleich 32 dem Betroffenen 6 mittels des Kopfhörers 12 dargeboten. Der Betroffene 6 vergleicht das Testsignal 12 mit dem Tinnitusgeräusch 4, insbesondere überprüft der Betroffene 6 hierbei, ob das Testsignal 18 das Tinnitusgeräusch 4 maskiert, das bedeutet überdeckt oder verdeckt. Mit anderen Worten bewertet der Betroffene 6, ob er das Tinnitusgeräusch 4 beim Einspielen des Testsignals 18 noch wahrnimmt. Das Testsignal 18 wird dem Betroffenen 6 hierbei ausreichend lang eingespielt beziehungsweise dargeboten, sodass er das Testsignal 18 zuverlässig mit dem Tinnitusgeräusch 4 vergleichen kann. Dies bedeutet, dass der Vergleich 32 im Wesentlichen eine Verdeckungsmessung des Tinnitusgeräuschs 4 mit dem Testsignal 18 ist.

Im Anschluss an ein negatives Vergleichsergebnis 20a, bei welchem das Tinnitusgeräusch 4 von dem Testsignal 18 nicht maskiert wird, oder im Anschluss an ein positives Vergleichsergebnis 20b, bei welchem das Tinnitusgeräusch 4 von dem Testsignal 18 maskiert wird, werden die Amplituden 30 der Schmalbandsignale 28 des Testsignals 18 in dem Verfahrensschritt 26 erneut zufällig oder auf Basis der bisherigen Ergebnisse variiert und ein Verfahrensschritt 34 gestartet.

In einem Speicher des Controllers 8 ist ein physiologisches Modell 36 einer Basilarmembran (Lamina basilaris oder Membrana basilaris) hinterlegt. Anhand des Modells 36 wird im Verfahrensschritt 34 für das jeweilige Testsignal 18 beziehungsweise dessen Schmalbandsignale 28 ein jeweiliges Erregungs- oder Anregungsmuster 38 (Fig. 3, Fig. 4) der Basilarmembran bestimmt. Die Berechnung oder Bestimmung des Anregungsmusters 38 berücksichtigt hierbei physiologische Faktoren, insbesondere auditorische Maskierungen, sowie beispielsweise einen Hörverlust oder Hörschaden des Betroffenen 6. Das Anregungsmuster 38 und das jeweilige Testsignal 18 werden anschließend in einem Speicher 40 hinterlegt. Aus den hinterlegten Testsignalen 18 und Anregungsmustern 38 des Speichers 40 wird in einem Verfahrensschritt 42 eine Wahrscheinlichkeits-Korrelation 44 zur Ermittlung des Tinnitusgeräuschs 4 bestimmt. Mit anderen Worten werden erfindungsgemäss mittels der Wahrscheinlichkeits-Korrelation 44 Gemeinsamkeiten der Testsignale 18 der positiven Vergleichsergebnisse 20b ermittelt, und somit eine Schätzung für das Testsignal 18 ermittelt, welches das Tinnitusgeräusch 4 am besten maskiert und somit am besten repräsentiert.

Der Vergleich 32 und die Verfahrensschritte 34 und 42 sowie das Hinterlegen des Testsignals 18 und der Anregungsmuster 38 im Speicher 40 bilden im Wesentlichen einen gemeinsamen Verfahrensschritt 46. Die Verfahrensschritte 46 und 26 werden hierbei wiederholt durchgeführt, bis die Wahrscheinlichkeits-Korrelation 44 bei einem Schwellwertvergleich 48 einen hinterlegten Schwellwert 50 erreicht oder überschreitet. Mit anderen Worten wird nach den Verfahrensschritten 26 und 46 der Schwellwertvergleich 48 durchgeführt und die Wahrscheinlichkeits-Korrelation 44 mit dem hinterlegten Schwellwert 50 verglichen.

Der Schwellwert 50 entspricht hierbei im Wesentlichen einer Mindestanzahl an positiven Vergleichsergebnissen 20b beziehungsweise einer Mindestanzahl an hinterlegten Testsignalen 18 und/oder Anregungsmustern 38, welche zur Bestimmung einer geeigneten Wahrscheinlichkeits-Korrelation 44 benötigt werden. Unter einer geeigneten Wahrscheinlichkeits-Korrelation 44 ist hierbei insbesondere eine Wahrscheinlichkeits-Korrelation 44 zu verstehen, welche eine gewisse Mindestwahrscheinlichkeit aufweist, dass ein daraus abgeleitetes Testsignal 18 das Tinnitusgeräusch 4 maskiert. Dies bedeutet, dass zu Beginn des Verfahrens die Amplituden 30 der Schmalbandsignale 28 des Testsignals 18 nach jedem Vergleich 32 zufällig eingestellt werden, wobei insbesondere für jedes positive Vergleichsergebnis 20b ein Anregungsmuster 38 berechnet und mit den Anregungsmustern 38 vorangegangener Vergleichsergebnisse 20a, 20b korreliert wird.

Bei einem negativen Vergleichsergebnis 52a des Schwellwertvergleichs 48, bei welchem die Wahrscheinlichkeits-Korrelation 44 den Schwellwert 50 nicht erreicht oder überschreitet, wird im Verfahrensschritt 26 ein neues Testsignal 18 mit zufälligen Amplituden 30 der Schmalbandsignale 28 erzeugt und dem Betroffenen 6 im Vergleich 32 eingespielt, und somit die Verfahrensschritte 26 und 46 wiederholt.

Bei einem positiven Vergleichsergebnis 52b des Schwellwertvergleichs 48, bei welchem der Schwellwert 50 von der Wahrscheinlichkeits-Korrelation 44 erreicht oder überschritten wird, wird ein zweiter Schwellwertvergleich 54 der Wahrscheinlichkeits-Korrelation 44 mit einem zweiten hinterlegten Schwellwert 56 durchgeführt. Der zweite Schwellwert 56 ist hierbei ein Wahrscheinlichkeitswert, welcher angibt, wie wahrscheinlich ein aus der Wahrscheinlichkeits-Korrelation 44 abgeleitetes Testsignal 18 das tatsächliche Tinnitusgeräusch 4 reproduziert. Mit anderen Worten ist der zweite Schwellwert 56 ein Maß inwieweit das aus Wahrscheinlichkeits-Korrelation 44 abgeleitetes Testsignal 18 dem Tinnitusgeräusch 4 entspricht. Der Schwellwert 56 ist beispielsweise auf eine Wahrscheinlichkeit von 90% dimensioniert. Erreicht oder Überschreitet Wahrscheinlichkeits-Korrelation 44 den Schwellwert 56 in einem positiven Vergleichsergebnis 58a, wird das Verfahren in einem Endschritt 60 beendet.

Im Falle eines negativen Vergleichsergebnisses 58b, bei welchem die Wahrscheinlichkeits-Korrelation 44 den Schwellwert 56 unterschreitet, wird ein Verfahrensschritt 62 gestartet. Im Verfahrensschritt 62 werden die Amplituden 30 der Schmalbandsignale 28 anhand der Wahrscheinlichkeits-Korrelation 44 eingestellt. Das dadurch erzeugte Testsignal 18 wird anschließend im Vergleich 32 dem Betroffenen 6 dargeboten.

Das Verfahren ist somit im Wesentlichen zweigeteilt. In einer Anfangsphase werden die Verfahrensschritte 26 und 46 wiederholt durchgeführt, wobei dem Betroffenen 6 jeweils ein Testsignal 18 mit zufällig eingestellten Amplituden 30 der Schmalbandsignale 28 dargeboten wird. Sind ausreichend positive Vergleichsergebnisse 20b vorhanden, also ausreichend viele Testsignale 18 und Anregungsmuster 38 im Speicher 40 hinterlegt, erreicht oder überschreitet die daraus bestimmte Wahrscheinlichkeits-Korrelation 44 den Schwellwert 50 im Schwellwertvergleich 48.

Nach dieser Anfangsphase startet mit dem Vergleichsergebnis 52b eine Endphase des Verfahrens, in welcher das Testsignal 18 mittels der Wahrscheinlichkeits-Korrelation 44 gezielt beeinflusst und eingestellt wird. In der Endphase werden anstelle der Verfahrensschritte 26 und 46 die Verfahrensschritte 62 und 46 wiederholt durchgeführt, und somit zunehmend Vergleichsergebnisse 20b beziehungsweise Testsignale 18 und Anregungsmuster 38 dem Speicher 40 zugeführt. Dadurch wird die Wahrscheinlichkeits-Korrelation 40 sukzessive verbessert, bis der Schwellwert 56 im Schwellwertvergleich 54 erreicht oder überschritten wird. Im Endschritt 60 wird das aus der Wahrscheinlichkeits-Korrelation 40 abgeleitete Testsignal 18 ausgegeben und/oder hinterlegt, dessen Schmalbandsignale 28 und Amplituden 30 im Wesentlichen den Amplituden 16 und Tinnitusfrequenzen 14 des Tinnitusgeräuschs 4 entspricht.

Die Figuren 3 und 4 zeigen jeweils ein Frequenz-Amplituden-Diagramm. Entlang der horizontalen x-Achse (Abszissenachse) des Diagramms ist die Frequenz f und entlang der vertikalen y-Achse (Ordinatenachse) des Diagramms ist die Amplitude A aufgetragen.

Die Fig. 3 und die Fig. 4 zeigen jeweils ein Testsignal 18 mit sieben Schmalbandsignalen 28, welche nachfolgend mit den Bezugszeichenzusätzen a bis g versehen sind, sowie das Tinnitusgeräusch 4, welches in diesem Ausführungsbeispiel lediglich durch eine Tinnitusfrequenz 14 mit der Amplitude 16 gebildet ist. Die Schmalbandsignale 28a bis 28g weisen jeweils die zugehörige Amplitude 30a bis 30g auf. Für die Schmalbandsignale 28e und 28f sowie 28g ist zusätzlich ein jeweiliges Anregungsmuster 38e, 38f, 38g punktiert eingezeichnet.

In den Ausführungsbeispielen der Fig. 3 und Fig. 4 ist das Tinnitusgeräusch 4 im Frequenzbereich des Schmalbandsignals 28f der Testsignale 18 angeordnet.

Das Testsignal 18 des Ausführungsbeispiels der Fig. 3 maskiert hierbei das Tinnitusgeräusch 4, da das Schmalbandsignals 28f eine größere Amplitude 30f aufweist als das Tinnitusgeräusch 4. Bei dem Vergleich 32 wird somit ein positives Vergleichsergebnis 20b bewirkt, und dadurch das Testsignal 18 sowie die entsprechenden Anregungsmuster 38 im Speicher 40 hinterlegt.

Das Testsignal 18 des Ausführungsbeispiels der Fig. 4 maskiert ebenfalls das Tinnitusgeräusch 4. Zwar weist das Schmalbandsignals 28f eine zu geringe Amplitude 30f auf um selbst das Tinnitusgeräusch 4 zu maskieren, jedoch bewirken die umliegenden Schmalbandsignale 28e und 28g eine auditorische Maskierung, sodass der Betroffene 6 das Tinnitusgeräusch 4 nicht wahrnimmt. Diese auditorische Maskierung wird hierbei durch die überlappenden Anregungsmuster 38e, 38f und 38g beschrieben. In diesem Ausführungsbeispiel wird die auditorische Maskierung insbesondere durch das Schmalbandsignal 28e beziehungsweise das Anregungsmuster 38e bewirkt. Somit wird auch für das Testsignal 18 der Fig. 4 im Vergleich 32 ein positives Vergleichsergebnis 20b bewirkt, und folglich das Testsignal 18 sowie die entsprechenden Anregungsmuster 38 im Speicher 40 hinterlegt.

Durch die zusätzliche Bestimmung und Berücksichtigung der Anregungsmuster 38 im Zuge der Bestimmung der Wahrscheinlichkeits-Korrelation 44 ist es somit möglich, breitbandige Testsignale 18 zu verwenden und hieraus sowohl die einzelnen Tinnitusfrequenzen 14 als auch deren Amplituden 16 des Tinnitusgeräuschs 4 zuverlässig zu bestimmen.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den in den Ansprüchen definierten Gegenstand der Erfindung zu verlassen.

In einer alternativen Ausgestaltung werden anstelle von Schmalbandsignalen 28 beispielsweise Tonkomplexe oder Tonkomponenten verwendet. Die Anzahl der Tonkomponenten hängt hierbei im Wesentlichen von der Intensität des zu erkennenden Tinnitus sowie gegebenenfalls von einem Hörverlust abhängigen Breite des Anregungsmusters auf der Basilarmembran ab. Ist zum Beispiel die Intensität des Tinnitus vergleichsweise gering und die Breite der Anregung der Basilarmembran vergleichsweise groß, kann das Verfahren mit einer reduzierten Anzahl von Tonkomponenten als Testsignal 18 durchgeführt werden.

Ein tonal wahrgenommener Tinnitus tritt hierbei häufig bei Frequenzen zwischen 1 kHz und 11 kHz, insbesondere zwischen 500 Hz und 11 kHz, auf. In einer vorteilhaften Weiterbildungsform ist die Bandbreite des Testsignals 18 zusätzlich oder alternativ anhand derartiger Auftretenswahrscheinlichkeiten reduzierbar.

### Bezugszeichenliste

- 2: Vorrichtung
- 4: Tinnitusgeräusch
- 6: Betroffener
- 8: Controller
- 10: Anzeigeeinheit
- 12: Kopfhörer
- 14: Tinnitusfrequenz
- 16: Tinnitusamplitude/Amplitude
- 18: Testsignal
- 20a, 20b: Vergleichsergebnis
- 22: Bilddaten
- 24: Start
- 26: Verfahrensschritt
- 28, 28a, 28b, 28c, 28d, 28e, 28f, 28g: Signalfrequenz/Schmalbandsignal
- 30, 30a, 30b, 30c, 30d, 30e, 30f, 30g: Signalamplitude/Amplitude
- 32: Vergleich
- 34: Verfahrensschritt
- 36: Modell
- 38, 38e, 38f, 38g: Anregungsmuster
- 40: Speicher
- 42: Verfahrensschritt
- 44: Wahrscheinlichkeits-Korrelation
- 46: Verfahrensschritt
- 48: Schwellwertvergleich
- 50: Schwellwert
- 52a, 52b: Vergleichsergebnis
- 54: Schwellwertvergleich
- 56: Schwellwert
- 58a, 58b: Vergleichsergebnis
- 60: Endschritt
- 62: Verfahrensschritt

- f: Frequenz
- A: Amplitude

## Patentansprüche

1. Verfahren zum Betreiben einer Vorrichtung (2) zur Tinnitus-Charakterisierung,
- bei dem in einem ersten Verfahrensschritt (46) ein breitbandiges Testsignal (18) mit einer Anzahl von Signalfrequenzen (28, 28a, 28b, 28c, 28d, 28e, 28f, 28g) erzeugt und mit einem Tinnitusgeräusch (4) hinsichtlich einer Maskierungswirkung verglichen wird, wobei das jeweilige Testsignal (18) mit einem zugeordneten Vergleichsergebnis (20b) hinterlegt wird, wobei bei einem positiven Vergleichsergebnis das Tinnitusgeräusch (4) maskiert wird, und wobei aus den hinterlegten Testsignalen (18) und Vergleichsergebnissen (20b) eine Wahrscheinlichkeits-Korrelation (44) zur Ermittlung der Gemeinsamkteiten der Testsignale (18) der positiven Vergleichsergebnisse bestimmt wird,
- bei dem in einem zweiten Verfahrensschritt (26) die Amplituden (30, 30a, 30b, 30c, 30d, 30e, 30f, 30g) der einzelnen Signalfrequenzen (28, 28a, 28b, 28c, 28d, 28e, 28f, 28g) des Testsignals (18) variiert werden,
- bei dem der erste und zweite Verfahrensschritt (46, 26) wiederholt durchgeführt werden, bis die Wahrscheinlichkeits-Korrelation (44) einen ersten Schwellwert (50) erreicht oder überschreitet, wobei der erste Schwellwert (50) im Wesentlichen einer Mindestanzahl von positiven Vergleichsergebnissen entspricht, welche eine Mindestwahrscheinlichkeit aufweisen, dass ein aus der Wahrscheinlichkeits-Korrelation (44) abgeleitetes Testsignal (18) das Tinnitusgeräusch (4) maskiert, und
- bei dem in einem dritten Verfahrensschritt (62) die Amplituden (30, 30a, 30b, 30c, 30d, 30e, 30f, 30g) der einzelnen Signalfrequenzen (28, 28a, 28b, 28c, 28d, 28e, 28f, 28g) des Testsignals (18) anhand der Wahrscheinlichkeits-Korrelation (44) variiert werden.

2. Verfahren nach Anspruch 1,
**dadurch gekennzeichnet,**
**dass** der erste und dritte Verfahrensschritt (46, 62) wiederholt durchgeführt werden, bis die Wahrscheinlichkeits-Korrelation (44) einen zweiten Schwellwert (56) erreicht oder überschreitet.

3. Verfahren nach Anspruch 1 oder 2,
**dadurch gekennzeichnet,**
**dass** die Amplituden (30, 30a, 30b, 30c, 30d, 30e, 30f, 30g) der einzelnen Signalfrequenzen (28, 28a, 28b, 28c, 28d, 28e, 28f, 28g) des Testsignals (18) im zweiten Verfahrensschritt (26) zufällig eingestellt werden.

4. Verfahren nach einem der Ansprüche 1 bis 3,
**dadurch gekennzeichnet,**
**dass** zur Bestimmung der Wahrscheinlichkeits-Korrelation (44) ein physiologisches Modell (36) für das jeweilige Testsignal (18) verwendet wird.

5. Verfahren nach Anspruch 4,
**dadurch gekennzeichnet,**
**dass** als physiologisches Modell (36) ein Modell einer Basilarmembran verwendet wird, wobei für das jeweilige Testsignal (18) ein jeweiliges Anregungsmuster (38, 38e, 38f, 38g) der modellierten Basilarmembran bestimmt und hinterlegt wird, und wobei die Wahrscheinlichkeits-Korrelation (44) anhand der hinterlegten Testsignale (18) und Anregungsmuster (38, 38e, 38f, 38g) bestimmt wird.

6. Vorrichtung (2) zur Tinnitus-Charakterisierung, aufweisend einen Signalgenerator zur Erzeugung eines akustischen Testsignals, und einen Hörer (12), mittels welchen das Testsignal einem Betroffenen (6) ausgebbar ist, sowie einen Controller (8) zur Durchführung eines Verfahrens nach einem der Ansprüche 1 bis 5.

## Claims

1. Method for operating a device (2) for tinnitus characterization,
- in which, in a first method step (46), a broadband test signal (18) with a number of signal frequencies (28, 28a, 28b, 28c, 28d, 28e, 28f, 28g) is generated and compared with a tinnitus noise (4) with regard to a masking effect, wherein the respective test signal (18) is stored with an associated comparison result (20b), wherein in case the comparison result is positive, the tinnitus noise is masked, and wherein a probability correlation (44) for determining the commonality of the test signals (18) of the positive comparison results is determined from the stored test signals (18) and comparison results (20b),
- in which, in a second method step (26), the amplitudes (30, 30a, 30b, 30c, 30d, 30e, 30f, 30g) of the individual signal frequencies (28, 28a, 28b, 28c, 28d, 28e, 28f, 28g) of the test signal (18) are varied,
- in which the first and second method step (46, 26) are repeatedly performed until the probability correlation (44) reaches or exceeds a first threshold value (50), wherein the first threshold value (50) substantially corresponds to a minimum number of positive comparison results having a minimum probability that a test signal (18) derived from the likelihood correlation (44) masks the tinnitus noise (4), and
- in which, in a third method step (62), the amplitudes (30, 30a, 30b, 30c, 30d, 30e, 30f, 30g) of the individual signal frequencies (28, 28a, 28b, 28c, 28d, 28e, 28f, 28g) of the test signal (18) are varied based on the probability correlation (44).

2. Method according to claim 1,
**characterized in**
**that** the first and third method step (46, 62) are repeatedly performed until the probability correlation (44) reaches or exceeds a second threshold value (56).

3. Method according to claim 1 or 2,
**characterized in**
**that** the amplitudes (30, 30a, 30b, 30c, 30d, 30e, 30f, 30g) of the individual signal frequencies (28, 28a, 28b, 28c, 28d, 28e, 28f, 28g) of the test signal (18) are randomly set in the second method step (26).

4. Method according to one of claims 1 to 3,
**characterized in**
**that** a physiological model (36) is used to determine the probability correlation (44) for the respective test signal (18).

5. Method according to claim 4,
**characterized in**
**that** a model of a basilar membrane is used as physiological model (36), wherein a respective excitation pattern (38, 38e, 38f, 38g) of the modeled basilar membrane is determined and stored for the respective test signal (18), and wherein the probability correlation (44) is determined based on the stored test signals (18) and excitation patterns (38, 38e, 38f, 38g).

6. Device (2) for tinnitus characterization, comprising a signal generator for generating an acoustic test signal, and a receiver (12), by means of which the test signal can be output to a person (6) affected, as well as a controller (8) for carrying out a method according to one of claims 1 to 5.

## Revendications

1. Procédé de fonctionnement d'un dispositif (2) de caractérisation des acouphènes,
- dans lequel, dans une première étape de procédé (46), un signal de test à large bande (18) avec un certain nombre de fréquences de signal (28, 28a, 28b, 28c, 28d, 28e, 28f, 28g) est généré et comparé à un bruit d'acouphène (4) en ce qui concerne un effet de masquage, dans lequel le signal de test respectif (18) est mémorisé avec un résultat de comparaison associé (20b), et dans lequel une corrélation de probabilité (44) pour déterminer le bruit d'acouphène (4) est déterminée à partir des signaux de test mémorisés (18) et des résultats de comparaison (20b),
- dans lequel, dans une deuxième étape de procédé (26), les amplitudes (30, 30a, 30b, 30c, 30d, 30e, 30f, 30g) des fréquences de signal individuelles (28, 28a, 28b, 28c, 28d, 28e, 28f, 28g) du signal de test (18) sont variées,
- dans lequel la première et la deuxième étape de procédé (46, 26) sont exécutées de manière répétée jusqu'à ce que la corrélation de probabilité (44) atteigne ou dépasse une première valeur de seuil (50), dans lequel la première valeur de seuil (50) correspond sensiblement à un nombre minimum de résultats de comparaison positifs ayant une probabilité minimum qu'un signal de test (18) dérivé de la corrélation de probabilité (44) masque le bruit d'acouphène (4), et
- dans lequel, dans une troisième étape de procédé (62), les amplitudes (30, 30a, 30b, 30c, 30d, 30e, 30f, 30g) des fréquences de signal individuelles (28, 28a, 28b, 28c, 28d, 28e, 28f, 28g) du signal de test (18) sont modifiées sur la base de la corrélation de probabilité (44).

2. Procédé selon la revendication 1,
**caractérisé en ce**
**que** la première et la troisième étape de procédé (46, 62) sont exécutées de manière répétée jusqu'à ce que la corrélation de probabilité (44) atteigne ou dépasse une seconde valeur de seuil (56).

3. Procédé selon la revendication 1 ou 2,
**caractérisé en ce**
**que** les amplitudes (30, 30a, 30b, 30c, 30d, 30e, 30f, 30g) des fréquences de signal individuelles (28, 28a, 28b, 28c, 28d, 28e, 28f, 28g) du signal de test (18) sont réglées de manière aléatoire dans la deuxième étape du procédé (26).

4. Procédé selon l'une des revendications 1 à 3,
**caractérisé en ce**
**qu'**un modèle physiologique (36) pour le signal de test respectif (18) est utilisé pour déterminer la corrélation de probabilité (44).

5. Procédé selon la revendication 4,
**caractérisé en ce**
**qu'**un modèle d'une membrane basilaire est utilisé comme modèle physiologique (36), dans lequel un modèle d'excitation respectif (38, 38e, 38f, 38g) de la membrane basilaire modélisée est déterminé et mémorisé pour le signal de test respectif (18), et dans lequel la corrélation de probabilité (44) est déterminée sur la base des signaux de test mémorisés (18) et des modèles d'excitation (38, 38e, 38f, 38g).

6. Dispositif (2) pour la caractérisation des acouphènes, comprenant un générateur de signaux pour générer un signal de test acoustique, et un récepteur (12), au moyen duquel le signal de test peut être délivré à une personne (6) concernée, ainsi qu'un contrôleur (8) pour exécuter un procédé selon l'une des revendications 1 à 5.
